# EUROPÄISCHE PATENTSCHRIFT

(11) **EP 0 509 308 B1**
(45) Veröffentlichungstag und Bekanntmachung des Hinweises auf die Patenterteilung: **23.11.1994**
(21) Anmeldenummer: 92105525.7
(22) Anmeldetag: 31.03.1992
(51) Int. Cl.: C07C 209/78, C07C 211/50

(54) **Verfahren zur Herstellung von mehrkernigen aromatischen Polyaminen**
Process for the preparation of polynuclear aromatic polyamines
Procédé pour la préparation de polyamines aromatique polynucléaires

(30) Priorität: 13.04.1991 DE 4112130
(43) Veröffentlichungstag der Anmeldung: 21.10.1992
(73) Patentinhaber: BAYER AG, 51368 Leverkusen (DE)
(72) Erfinder: Knöfel, Hartmut, Dr., W-5068 Odenthal 3 (DE); Brockelt, Michael, W-5090 Leverkusen 3 (DE); Petinaux, Marcel, Dr., Pittsburgh, PA 15241 (US); Uchdorf, Rudolf, Dipl.-Ing., W-4150 Krefeld 11 (DE); Schal, Hans-Peter, Dr., W-4047 Dormagen 1 (DE)

(56) Entgegenhaltungen:
- EP-A- 0 288 892
- EP-A- 0 337 205

## Beschreibung

Die Erfindung betrifft eine verbesserte Ausführungsform des Verfahrens der Herstellung von mehrkernigen aromatischen Polyaminen durch Kondensation von Anilin mit Formaldehyd in Gegenwart von Wasser und sauren Katalysatoren und Aufarbeitung des Reaktionsgemisches durch Extraktion mit einem hydrophoben Lösungsmittel unter Wiederverwendung des in der wäßrigen Phase der Extraktion anfallenden Säurekatalysators.

Es ist bereits bekannt, bei der Herstellung von mehrkernigen aromatischen Polyaminen durch Kondensation von Anilin mit Formaldehyd in Gegenwart von Wasser und sauren Katalysatoren das anfallende wäßrige Reaktionsgemisch durch Extraktion mit einem hydrophoben Lösungsmittel aufzuarbeiten und den bei der Extraktion in der wäßrigen Phase anfallenden Säurekatalysators wiederzuverwenden (vgl. z.B. DE-OS 2 238 920, DE-OS 2 343 658, DE-OS 2 356 828, DE-OS 2 500 573, DE-OS 2 500 574 oder DE-OS 2 528 694).

Der wesentliche Vorteil der Verfahren dieser Vorveröffentlichungen ist darin zu sehen, daß auf eine Neutralisation des Katalysators verzichtet werden kann, da er bei der extraktiven Aufarbeitung des sauren Reaktionsgemischs in der wäßrigen Phase anfällt, die als solche an den Prozeßanfang zurückgeführt und wiederverwendet wird. Außerdem gestatten bestimmte Varianten dieses bekannten Prinzips, wie sie beispielsweise in DE-OS 2 500 574 oder 2 528 694 beschrieben sind, die gezielte Herstellung von Polyamingemischen mit wahlweise erhöhtem oder erniedrigtem Gehalt an 2,4'-Isomeren. Im übrigen entsprechen die Verfahrensprodukte der Vorveröffentlichungen bezüglich ihrer Eignung als Vorprodukt zur Herstellung von Polyisocyanaten den klassischen Polyaminen der Diphenylmethanreihe, die unter Neutralisation des eingesetzten Säurekatalystors hergestellt worden sind. Dies bedeutet, daS das Eigenschaftsniveau der aus derartigen Polyisocyanatgemischen der Diphenylmethanreihe hergestellten Polyurethanschaumstoffe in beiden Fällen annähernd das gleiche ist. Es ist aber als ein Nachteil der Verfahren der genannten Vorveröffentlichungen anzusehen, daß zur Aufarbeitung der Verfahrensprodukte durch Extraktion ausschließlich für diesen Zweck beträchtliche Mengen an hydrophobem Lösungsmittel und Anilin eingesetzt werden müssen, was selbstverständlich auf einen beträchtlichen Destillationsaufwand und damit Energieverbrauch bei der destillativen Aufarbeitung der organischen Phase hinausläuft.

Einen gewissen Fortschritt gegenüber dem o.g. Stand der Technik unter Erhaltung von dessen Vorteilen stellen später veröffentlichte Verfahren des Standes der Technik dar, wie sie in EP 0 288 892 und EP 0 337 205 beschrieben dar.

Die Vorteile dieser Verfahren betreffen insbesondere die Produktqualität und Produktflexibilität neben ökonomischen Verbesserungen.

Sie beruhen im wesentlichen auf der Mitverwendung eines hydrophoben Lösungsmittels auch wahrend des Reaktionsablaufs, Dabei resultieren weitere Vorteile aus der teilweisen Doppelfunktion dieses hydrophoben Lösungsmittels als Bestandteil sowohl des Reaktionsgemisches als auch des Extraktionsmittels.

Insgesamt zeichnet sich der Stand der Technik gemäß EP 0 288 892 und EP 0 337 205 durch folgende Vorteile aus:
- Der eingesetzte Saurekatalysator wird wiederverwendet und nicht durch Neutralisation vernichtet.
- Die bei der destillativen Aufarbeitung der, die Verfahrensprodukte enthaltenden, organischen Phase als Destillat anfallenden Gemische, können ohne weitere destillative Auftrennung in ihre Bestandteile als solche, gegebenenfalls nach Zugabe von weiterem Anilin, als Extraktionsmittel für die wäßrige Phase in der Produktextraktionsstufe wiederverwendet werden.
- Die Verfahren sind bezüglich der Homologenverteilung in den Verfahrensprodukten (Verhältnis von Diaminen zu höherfunktionellen Polyaminen) innerhalb weiter Grenzen variierbar.
- Die Verfahren gestatten insbesondere die Herstellung von Polyaminen der Diphenylmethanreihe mit einem geringen Gehalt an 2,4'-Diaminodiphenylmethan bei gleichzeitig geringem Gehalt an stets unerwünschtem 2,2'-Diaminodiphenylmethan.
- Die aus den Verfahrensprodukten hergestellten Polyisocyanate liefern überraschenderweise Polyurethanschaumstoffe, die im Vergleich zu entsprechenden Polyurethanschaumstoffen auf Basis der älteren bekannten Polyisocyanatgemische der Diphenylmethanreihe eine deutlich geringere Eigenfarbe aufweisen,
   Bei den Verfahren kann die Gesamtmenge des Lösungsmittels wesentlich reduziert werden, so daß die MDA-Konzentration in den anfallenden organischen Phasen beträchtlich erhöht und damit der Destillationsaufwand bei der Aufarbeitung der organischen Phasen entsprechend erniedrigt werden kann.

Als Nachteil dieser Verfahren erwiesen sich die im Zusammenhang mit der vorteilhaften hohen Selektivität der zweiphasigen Reaktionsführung einhergehenden verlängerten Reaktionszeiten, insbesondere in der zweiten Umlagerungsstufe, bis zur vollständigen Umlagerung in die gewünschten Endprodukte.

Daraus resultiert die Gefahr der unvollständigen Umlagerung mit der sich ergebenen Problematik für die Qualität der Endprodukte und insbesondere der abgeleiteten Polyisocyanate.

Unvollständige Umlagerung bedeutet aber das Vorhandensein von Kondensationszwischenprodukten z.B. vom N-Aminobenzyltyp im Reaktionsgemisch am Ende der eigentlichen Reaktion. Bei der Aufarbeitung des Reaktionsgemisches zur Isolierung der Verfahrensendprodukte gemäß den Verfahren des Standes der Technik gelangen die Zwischen- und Nebenprodukte in die Verfahrensendprodukte, was mit deutlichen Qualitätseinbußen insbesondere bei den resultierenden Polyisocyanaten verbunden ist, Um letzteres durch Sicherstellung von vollständiger Umlagerung am Ende der Reaktion zu vermeiden, muß bei den zitierten Verfahren des Standes der Technik, insbesondere bei kontinuierlicher Betriebsweise, mit einem erheblichen Aufwand bei der Reaktionszeit bzw. dem Reaktionsvolumen und/oder der Reaktionstemperatur, insbesondere in der letzten Umlagerungsstufe, gegengesteuert werden.

Bei EP 0 288 892 wird die organische Phase am Ende der Umlagerungsreaktion abgetrennt und zur Gewinnung der Verfahrensendprodukte mitsamt evtl. vorhandener Kondensationszwischenprodukte eingesetzt.

Letztere sind in dieser organischen Phase (E) gegenüber der wäßrigen Phase (F) angereichert und gelangen mit dieser über die Aufarbeitungsstufe(n) in das Verfahrensprodukt.

Bei EP 0 337 205 werden aus der organischen Phase nach ihrer Abtrennung am Ende der Umlagerungsreaktion die darin enthaltenen Kondensationsprodukte insgesamt durch Extraktion in eine wäßrige Phase überführt und in den Reaktionsablauf rezykliert.

Die verbleibende organische Phase wird anschließend an anderer Stelle als Extraktionsmittel für die Verfahrensprodukte eingesetzt. Bei der Extraktion mit der wäßrigen Phase konzentrieren sich evtl. vorhandene Kondensationszwischenprodukte neben u.a. dem 2,2'-Isomeren des Diaminodiphenylmethans zunächst in dieser organischen Phase infolge der Selektivität dieses Vorgangs auf, um schließlich als letzte Komponenten des Kondensationsproduktgemisches in die wäßrige Phase überzugehen. Die Extraktion muß daher mit erheblichen Aufwand möglichst quantitativ durchgeführt werden, damit die verbleibende organische Phase als Extraktionsmittel für Verfahrensendprodukte in der Hauptextraktionsstufe ohne Nachteil nochmals verwendet werden kann.

Es war die der Erfindung zugrunde liegende Aufgabe, ein neues verbessertes Verfahren zur Herstellung von mehrkernigen aromatischen Polyaminen aus Anilin und Formaldehyd zur Verfügung zu stellen, welches die Vorteile des bekannten Standes der Technik in sich vereinigt und unter Vermeidung der Nachteile die Herstellung von qualitativ weiter verbesserten Produkten bei gleichzeitig reduziertem Verfahrensaufwand und verbesserter Sicherheit bei der erzielten Produktqualität ermöglicht.

Diese Aufgabe konnte mit dem nachstehend näher beschriebenen Verfahren gelöst werden.

Durch selektive Abtrennung und interne Rückführung von Kondensationszwischenprodukten in den Reaktionsablauf vor der Gewinnung und Isolierung der eigentlichen Verfahrensprodukte ergaben sich folgende Verbesserungen:
1. Die isolierten Verfahrensprodukte weisen einen äußerst geringen Gehalt an Kondensationszwischenprodukten auf, der in allen Fällen deutlich geringer ist als in den Reaktionsprodukten am Ende der eigentlichen Reaktion, daher kann
2. in den Reaktionsendprodukten ein höherer Gehalt an Kondensationszwischenprodukten toleriert werden als bei den Verfahren des Standes der Technik ohne Beeinträchtigung der Verfahrensendprodukte bzw. der Folgeprodukte.
3. Darüber hinaus bietet das erfindungsgemäße Verfahren zusätzliche Sicherheit für die Qualität der Endprodukte gegen "Durchschlagen" von Kondensationszwischenprodukten in die isolierten Endprodukte, falls durch Verfahrensschwankungen und -störungen am Ende der Umlagerungsreaktion erhöhte Werte dieser Zwischenprodukte auftreten.
4. Die isolierten Verfahrensprodukte nach der Aufarbeitung weisen stets einen geringeren Gehalt an 2,2'-Diaminodiphenylmethan auf als die Reaktionsendprodukte
5. Die isolierten Verfahrensendprodukte weisen gegebenenfalls auch einen geringeren Gehalt an 2,4'-Diaminodiphenylmethan auf als die Reaktionsendprodukte.
6. Der Gehalt der Verfahrensendprodukte an 2,4'-Diaminodiphenylmethan kann in weiten Grenzen gesteuert werden, unabhängig von den eigentlichen Reaktionsparametern wie Anilin-Formaldehyd-(=Kondensations-)verhältnis, Amin-Katalysatorverhältnis (=Protonierungsgrad), Phasenverhältnis von organischer zu wäßriger Phase während der Reaktion.
7. Die isolierten Verfahrensprodukte weisen gegebenenfalls auch einen geringeren Gehalt an ortho-substituierten höheren Homologen des Diamindiphenylmethans auf als die Reaktionsendprodukte.

Gegenstand der Erfindung ist ein Verfahren zur Herstellung von mehrkernigen aromatischen Polyaminen durch Umsetzung von Anilin mit Formaldehyd in Gegenwart von Wasser und sauren Katalysatoren in einer ein- oder zweistufigen Reaktion innerhalb des Temperaturbereichs von 0 bis 180°C, gegebenenfalls unter Vorschaltung einer Aminal-Vorstufe, in welcher die Bildung von N,N'-disubstituiertem Aminal stattfindet, welches dann ein- oder mehrstufig in Gegenwart von Saurekatalysator innerhalb des Temperaturbereichs von 0 bis 180°C in das gewünschte Endprodukt überführt wird, Aufarbeitung des resultierenden Reaktionsgemischs und Gewinnung der resultierenden Verfahrensendprodukte durch Extraktion mit einem Anilinhaltigen hydrophoben Losungsmittel in einer Produktextraktionsstufe (8) destillative Auftrennung der hierbei anfallenden organischen Phase in (i) ein aus Anilinhaltigem Lösungsmittel bestehendes Destillat, welches gegebenenfalls nach Zugabe von Frischanilin erneut bei der Extraktion eingesetzt wird und (ii) einen im wesentlichen aus Verfahrensendprodukt bestehenden Destillationsrückstand und Rückführung von den Säurekatalysator enthaltender wäßriger Phase unter Wiederverwendung des in ihr enthaltenen Katalysators, unter Entfernung des bei der Kondensationsreaktion entstehenden Kondensationswassers und des in das System mit der wäßrigen Lösung des Formaldehyds eingebrachten Wassers in einem der Aminalvorstufe nachgeschalteten und der ersten Reaktionsstufe vorgeschalteten Wasserabscheider und/oder in einem der Extraktionsstufe nachgeschalteten Verdampfer, dadurch gekennzeichnet, daß man
a) den in Form einer wäßrigen Losung zum Einsatz gelangenden Formaldehyd in einer Aminal-Vorstufe (3) mit einer aus Anilin, gegebenenfalls Anilin-Formaldehyd-Kondensationsprodukten und aus hydrophobem Lösungsmittel bestehenden organischen Phase und/oder in der ersten Reaktionsstufe (5) mit einer aus Anilin, gegebenenfalls Anilin-Formaldehyd-Kondensationsprodukten und aus hydrophobem Lösungsmittel bestehenden organischen Phase und recyclisierter, den Katalysator in Form von Aminsalzen enthaltender wäßriger Phase durch Vermischen zur Reaktion bringt,
b) das so erhaltene zweiphasige Reaktionsgemisch nach Beendigung der Reaktion in einem der Produktextraktionsstufe (8) vorgeschalteten Phasenscheider (7) in eine wäßrige Phase und eine organische Phase auftrennt,
c) die im Phasenscheider (7)anfallende organische Phase in einer der Produktextraktionsstufe (8) nachgeschalteten Nachextraktionsstufe (10) mit wenigstens einem Teil der in der Produktextraktionsstufe (8) anfallenden, weitgehend vom Reaktionsprodukt befreiten wäßrigen Phase extrahiert,
d) die in der Nachextraktionsstufe (10) anfallende mit Reaktionsprodukt der im Phasenscheider (7) anfallenden organischen Phase angereicherten wäßrigen Phase vollständig oder zumindest teilweise in den Reaktionsprozess vor beendeter Umlagerung (6) zurückführt und den gegebenenfalls verbleibenden Rest der wäßrigen Phase zwischen die Umlagerungsstufe (6) und die Hauptextraktionsstufe (8) zurückführt,
e) die in der Nachextraktionsstufe (10) anfallende, aus Anilin, gegebenenfalls Anilin-Formaldehyd-Kondensationsprodukten und aus hydrophobem Lösungsmittel bestehende organische Phase an den Prozessanfang zurückführt und gemäß a) umsetzt,
f) die im Phasenscheider (7) anfallende wäßrige Phase in der Produktextraktionsstufe (8) mit Anilinhaltigem, hydrophoben Lösungsmittel extrahiert,
g) die in der Produktextraktionsstufe (8) anfallende wäßrige Phase vollständig oder zumindest teilweise der Nachextraktionsstufe (10) zuführt und gegebenenfalls den verbleibenden Rest vor die Umlagerungsstufe (6), vorzugsweise an den Prozeßanfang zurückführt,
h) die in der Produktextraktionsstufe (8) anfallende organische Phase in der Destillationsstufe (11) in ein aus Anilin-haltigem hydrophoben Lösungsmittel bestehendes Destillat und einen im wesentlichen aus Verfahrensendprodukt bestehenden Destillationsrückstand auftrennt und
i) das in der Destillationsstufe (11) anfallende Destillat gegebenenfalls nach Zugabe von Frischanilin in der Produktextraktionsstufe (8) als Extraktionsmittel verwendet.

Ausgangsmaterialien für das erfindungsgemäße Verfahren sind Anilin und Formaldehyd oder formaldehydabspaltende Mittel. Der Formaldehyd wird vorzugsweise in Form einer wäßrigen Lösung mit einem Formaldehydgehalt von 20 bis 50 Gew.-% eingesetzt.

Bei den zum Einsatz gelangenden hydrophoben Lösungsmitteln handelt es sich um inerte Lösungsmittel des Siedepunktbereichs 30-250°C, vorzugsweise 80-200°C wie
beispielsweise Chlorbenzol, Dichlorbenzole, Benzol, Toluol, Xylol, Dichlorethan, Chloroform oder Tetrachlorkohlenstoff. Vorzugsweise werden Xylole, d.h. technische Xylolgemische und insbesondere o-Xylol als hydrophobes Lösungsmittel verwendet,

Bei dem Säurekatalysator handelt es sich um wasserlösliche Säuren mit einem unter 2,5, vorzugsweise unter 1,5 liegendem pKa-Wert. Beispiele hierfür sind Salzsäure, Bromwasserstoffsäure, Schwefelsäure, Trifluoressigsäure, Methansulfonsäure oder Phosphorsäure. Bevorzugt einzusetzender Katalysator ist Salzsäure. Die genannten Säuren können auch im Gemisch mit sauren oder neutralen Salzen derartiger Säuren, wie z.B. den entsprechenden Ammoniumsalzen oder auch den entsprechenden Alkalisalzen eingesetzt werden; die Mitverwendung derartiger Salze ist jedoch weniger bevorzugt. Die genannten Säuren liegen in dem erfindungsgemäßen Kreislaufsystem in Form der entsprechenden Ammoniumsalze der im wäßrigen Kreislauf befindlichen Basen vor.

Das erfindungsgemäße Verfahren kann sowohl ein- als auch zweistufig und sowohl unter Vorschaltung einer Aminal-Vorstufe als auch ohne eine derartige Aminal-Vorstufe durchgeführt werden, wobei jedoch einschränkend hinzugefügt werden muß, daß bei einstufiger Reaktionsführung die Vorschaltung einer Aminal-Vorstufe auf jeden Fall angezeigt ist.

Unter "einstufiger Reaktionsführung" ist hierbei eine Verfahrensvariante zu verstehen, gemäß welcher das Aminal nach Hinzufügung des Säuekatalysators innerhalb eines kurzen Zeitraums von weniger als 10 Minuten, vorzugsweise von weniger als 5 Minuten auf eine erhöhte Temperatur von 60 bis 180, vorzugsweise 80 bis 150°C erhitzt wird, um dort in das Endprodukt umlagert zu werden, bzw. bei welcher das Aminal direkt mit der auf erhöhtem Temperaturniveau von 60-180°C, vorzugsweise 80 bis 150°C befindlichen und im Kreis geführten wäßrigen Katalystorphase vermischt und das Gemisch dann gegebenenfalls bis zur angestrebten Endtemperatur erhitzt wird.

Unter "zweistufiger Reaktionsführung" ist eine Ausführungsform zu verstehen, gemäß welcher das Aminal nach Zugabe des Säurekatalysators oder das Reaktionsgemisch aus Anilin, gegebenenfalls Anilinformaldehydkondensaten, Formaldehyd und Säurekatalysator zunächst während eines Zeitraums von 10 bis 90, vorzugweise 15 bis 60 Minuten in einer ersten Reaktionsstufe bei 0 bis 60°C, vorzugsweise 30 bis 60°C und anschließend während eines Zeitraums von 30 bis 180, vorzugsweise 30 bis 120 Minuten in einer zweiten Reaktionsstufe bei 60 bis 180°C, vorzugsweise 60 bis 150°C und insbesondere 90 bis 145°C gehalten wird. Bei dieser bevorzugten Verfahrensvariante einer mehrstufigen, vorzugsweise zweistufigen Reaktionsführung erfolgt in der ersten Reaktionsstufe vor allem eine Umlagerung des Aminals oder (im Falle eines Verzichts auf eine Aminalvorstufe) eine Kondensation der Ausgangsmaterialien zu Produkten von N-Benzyl-arylamintyp, welches in der zweiten Reaktionsstufe bei erhöhter Temperatur zum kernsubstituierten Endprodukt umgelagert werden. Gemäß einer besonderen Ausführungsform mit zweistufiger Reaktionsführung - ohne oder vorzugsweise mit Aminalvorstufe - wird die erste Reaktionsstufe nur mit einem Teilstrom der wäßrigen Katalysatorphase, im allgemeinen < 50 %, vorzugsweise < 15 %, eingeleitet. In weiteren Verlauf der ersten Reaktionsstufe und vor Beendigung der letzten, d. h. im allgemeinen zweiten Reaktionsstufe erfolgt die Vervollständigung der Reaktion in Gegenwart der gesamten Katalysatorphase.

Das Verfahren kann sowohl kontinuierlich als auch diskontinuierlich durchgeführt werden; die angegebenen Zeiträume beziehen sich bei kontinuierlicher Fahrweise auf die mittlere Verweilzeit des Reaktionsgemisches in den einzelnen Stufen. Im Falle der Vorschaltung einer Aminal-Vorstufe beträgt die (mittlere) Verweilzeit der Ausgangsmaterialien in dieser Stufe im allgemeinen 10 bis 60, vorzugsweise 15 bis 60 Minuten. Die Temperatur in der Aminal-Vorstufe liegt im allgemeinen bei 20 bis 100°C, vorzugsweise 20 bis 60°C. Das Verfahren wird in allen Stufen vorzugsweise unter dem Eigendruck des Systems und vorzugsweise in einer Intergasatmosphäre (Stickstoff) durchgeführt.

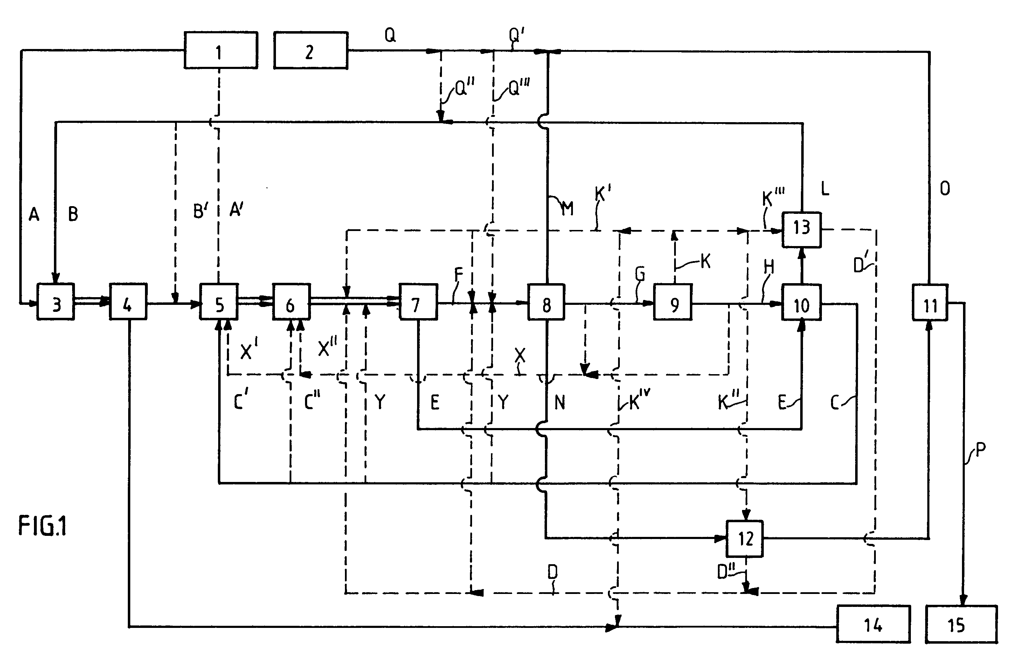

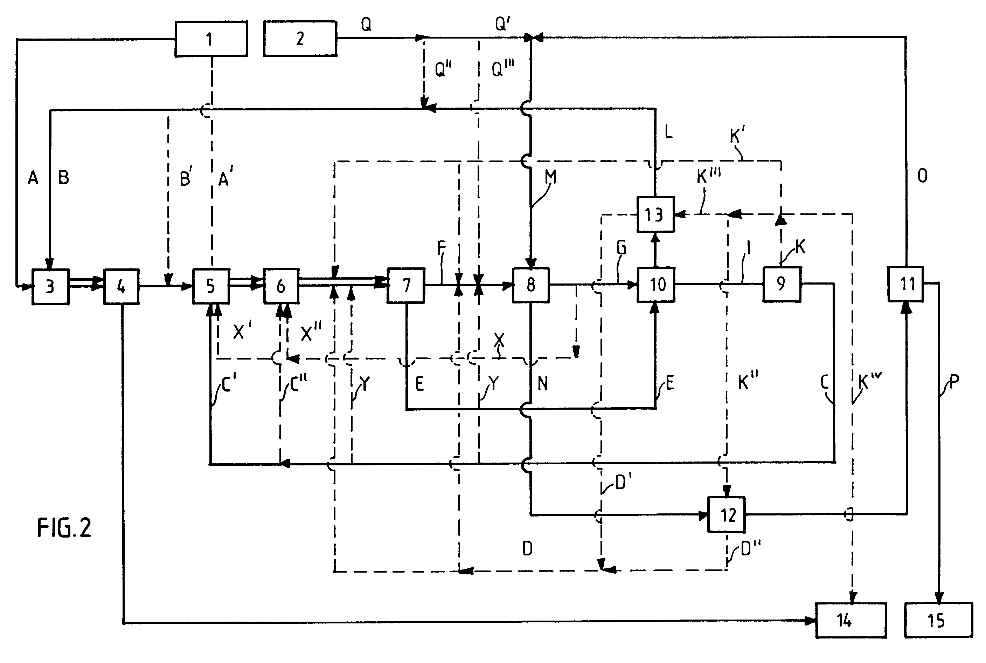

Die in den Figuren 1 und 2 dargestellten Fließdiagramme dienen der weiteren Erlauterung des erfindungsgemäßen Verfahrens. In diesen Abbildungen bedeuten:
(1) einen Tank für wäßrige Formaldehydlösung
(2) einen Tank für Anilin
(3) einen Kondensationsreaktor (Aminal-Vorstufe)
(4) einen Wasserabscheider
(5) die erste Reaktionsstufe
(6) die zweite Reaktionsstufe
(7) einen Phasenscheider
(8) die Produktextraktionsstufe
(9) einen Wasserverdampfer
(10) die Nachextraktionsstufe
(11) die Produktdestillationsstufe
(12) eine Waschstufe
(13) eine Waschstufe
(14) einen Tank für Abwasser
   und
(15) einen Tank für Verfahrensprodukt.

Die Bezugszeichen A bis Q, X und Y bezeichnen die Mengenströme, auf die nachstehend und in den Beispielen Bezug genommen wird.

Im Falle der genannten einstufigen Reaktionsführung werden die Reaktionsstufen (5) und (6) zu einer einzigen Reaktionsstufe zusammengefaßt. Sowohl die erste als auch die zweite Reaktionsstufe können sowohl aus einem einzelnen als auch aus mehreren, in Serie geschalteten Reaktoren bestehen. Zur Aufrechterhaltung der genannten Verweilzeiten haben sich insbesondere in Serie geschaltete Rührkesselkaskaden und oder Kolonnenreaktoren bewährt.

Auch die Extraktionsstufe kann aus einem oder mehreren, in Serie geschalteten Extraktoren bestehen, Hier werden vorzugsweise die üblichen Gegenstromextraktionsvorrichtungen verwendet.

Die Destillationsstufe (11) besteht im einfachsten Fall aus einer Destillsationskolonne, die so ausgelegt ist, daS eine weitgehende Abtrennung von hydrophobem Lösungsmittel und Anilin vom Verfahrensprodukt möglich ist.

Als ein besonderer Vorteil des erfindungsgemäßen Verfahrens ist dabei der Umstand anzusehen, daß die Auftrennung von hydrophobem Lösungsmittel und Anilin nicht erforderlich ist, da der Anilingehalt im Destillat in der Regel nicht über dem für die Wiederverwendung erforderlichen Wert liegt, der vor der Wiederverwendung je nach Bedarf durch Zugabe von frischem Anilin eingestellt wird, und so der Einsatz energiesparender Mehrstufendestillationstechniken zur Bewältigung der Destillationsaufgabe ermöglich wird.

Die Destillationsstufe (11) wird daher vorzugsweise mehrstufig betrieben, indem unter maximaler Ausnutzung der aufgewendeten Destillationsenergie in einer ersten Destillationsstufe eine Destillatfraktion erhalten wird, die das niedriger siedende hydrophobe Lösungsmittel in relativ angereicherter Form neben Anilin in relativ abgereicherter Form enthält und in einer letzten Destillationsstufe eine Destillatfraktion erhalten wird, die das hydrophobe Lösungsmittel in relativ abgereicherter und das Anilin in relativ angereicherter Form enthält. Bevorzugt ist eine Ausführungsform, bei der in einer letzten Destillationsstufe als Destillat Anilin nahezu frei von hydrophoben Lösungsmittel erhalten wird.

Das bei der Kondensation entstehende und das mit der wäßrigen Formaldehydlösung in das System eingebrachte Wasser muß an einer geeigneten Stelle zwecks Aufrechterhaltung eines konstanten Wasservolumens aus dem System entfernt werden. Bei Vorschaltung einer Aminalvorstufe (3) geschieht diese Wasserentfernung vorzugsweise im Wasserabscheider (4) bevor das Aminal mit dem Säurekatalysator zusammengebracht wird. In Abwesenheit einer Aminalvorstufe geschieht die Entfernung des Wassers vorzugsweise in einem Wasserverdampfer (9), der nach der Produktextraktionsstufe (8) angeordnet ist. Dieser Wasserverdamper wird vorzugsweise nach dem Prinzip der Entspannungsverdampfung durch Anlegen von Vakuum betrieben.

Grundsätzlich ist es jedoch auch möglich, dem System Wasser an einer beliebigen andern Stelle durch Destillation zu entnehmen.

Bei der Durchführung des erfindungsgemäßen Verfahrens sind mehrere Varianten möglich, die nachstehend im Detail beschrieben werden.

Gemäß einer 1. Variante des erfindungsgemäßen Verfahrens erfolgt die Einspeisung der wäßrigen Formaldehydlösung (A) in die Aminalstufe (3), in welcher die Umsetzung mit einem Gemisch (B) aus Anilin, gegebenenfalls Anilin-Formaldehyd-Kondensationsprodukten und hydrophobem Lösungsmittel, stattfindet. Bei dem Mengenstrom (B) handelt es sich im wesentlichen um die die Nachextraktionsstufe (10) bzw. die Waschstufe (13) verlassende organische Phase, der gegebenenfalls Anilin aus dem Vorratstank (2) zugesetzt wird.

Das Molverhältnis von Anilin zu Formaldehyd liegt im allgemeinen in der Aminalstufe zwischen 1,5:1 und 25:1, vorzugsweise bei 1,8:1 bis 10:1.

Das Gewichtsverhältnis von Arylamin ( = Anilin sowie gegebenenfalls vorliegende Anilin-Formaldehyd-Kondensationsprodukte) zu hydrophobem Losungsmittel in (B) liegt im allgemeinen zwischen 1:4 und 3:1, vorzugsweise zwischen 1:2 und 2:1.

Die Umsetzung in der Aminalstufe (3) erfolgt innerhalb der obengenannten Temperaturbereiche.

Im Anschluß an (3) erfolgt in einem Scheider (4) die mechanische Abtrennung der wäßrigen Phase, welche von dem Kondensationswasser und dem Formalinwasser gebildet wird und daneben die wasserlöslichen Verunreinigungen des Formaldehyds und des Mengenstroms (B) enthält. Die Trennung erfolgt vorzugsweise unterhalb 60°C.

Die verbleibende organische Phase wird in den Reaktor (5) übergeführt und bei Temperaturen unterhalb von 60°C mit dem wäßrigen Mengenstrom (C') zusammengebracht.

Bei dem Mengenstrom (C') handelt es sich bei dieser ersten Variante des erfindungsgemäßen Verfahrens um die Gesamtmenge der zurückzuführenden Katalysatorphase (C). Der Gehalt an Anilin-Formaldehyd-Kondensaten in dieser Phase (C) liegt im allgemeinen bei 10 bis 40 Gew.-%, vorzugsweise 15 bis 30 Gew,-% bei einem Gesamtgehalt an Arylamin (inklusive Anilin) an dieser Stelle im allgemeinen von 30-70 Gew.-%, vorzugsweise 40-60 Gew.-%, und bei einem Protonierungsgrad von 25 bis 75, vorzugsweise 45 bis 70 %. Unter "Protonierungsgrad" ist hierbei und auch nachstehend der Prozentsatz der Aminstickstoffatome zu verstehen, die in Form von Ammoniumgruppen, d.h. "protoniert" vorliegen.

Das Gewichtsverhältnis zwischen organischer Phase aus (4) und Katalysatorphase (C) liegt im allgemeinen zwischen 100:1 und 1:10, vorzugsweise zwischen 4:1 und 1:3.

Bei der ersten, kontinuierlich betriebenen Variante des erfindungsgemäßen Verfahrens stellt der Reaktor (5) die bereits obengenannte "erste Reaktionsstufe" dar, die unter den obengenannten Bedingungen bezüglich der Temperatur und der Reaktionszeit betrieben wird. Im allgemeinen handelt es sich um eine mehrstufige Rührkesselkaskade oder einen ein- oder mehrstufigen Kolonnenreaktor, in welchem vorzugsweise ein Temperaturprofil von ca. 20°C am Beginn ansteigend bis auf 60°C am Ende durchlaufen wird.

Aus der ersten Reaktionsstufe (5) wird das zweiphasige Reaktionsgemisch in die zweite Reaktionsstufe (6) überführt, die ebenfalls aus einer mehrstufigen Rührkesselkaskade oder einem ein- oder mehrstufigen Kolonnenreaktor besteht. Diese zweite Reaktionsstufe wird ebenfalls unter den bereits obengenannten Bedingungen bezüglich der Reaktionstemperatur und der mittleren Verweilzeit betrieben. Vorzugsweise durchlauft das zweiphasige Reaktionsgemisch in der Reaktionsstufe (6) ein Temperaturprofil beginnend bei 60°C und endend bei einer Temperatur zwischen 90 und 150°C, vorzugsweise zwischen 95 und 140°C. Im Falle dieser bevorzugten Temperaturführung sind in der Reaktionsstufe (6) im allgemeinen Verweilzeiten von bis zu 60 Minuten ausreichend.

Das die zweite Reaktionsstufe (6) verlassende zweiphasige Reaktionsgemisch wird anschließend im Phasenscheider (7), vorzugsweise bei Temperaturen zwischen 80 und 110°C in eine organische Phase (E) und eine wäßrige Phase (F) zerlegt, die gegebenenfalls mit dem Strom (Y) vereinigt und der Produktextraktionsstufe (8) zugeführt wird.

Aus der wäßrigen Phase (F) werden in dem vorzugsweise mehrstufig wirkenden Extraktor (8), der vorzugsweise bei Temperaturen von 80 bis 110°C betrieben wird, die Verfahrensprodukte im Austausch gegen Anilin extrahiert und in eine organische Lösung (N) überführt.

Als Extraktionsmittel (M) dient ein Gemisch aus hydrophobem Lösungsmittel und Anilin. Das Gewichtsverhältnis Anilin : Lösungsmittel liegt im allgemeinen zwischen 0,5:1 und 3:1, vorzugsweise 1:1 und 2:1.

Das Gewichtsverhältnis Extraktionsmittel (M) : wäßrige Phase in der Extraktionsstufe (8) liegt im allgemeinen zwischen 0,5:1 und 3:1, vorzugsweise bei 0,7:1 bis 2:1.

Die organische Phase (N) wird, gegebenenfalls nach Durchlaufen der Katalysatorwaschstufe (12), in der gegebenenfalls Katalysatorspuren entfernt werden, in die Destillationsstufe (11) überführt.

In der Destillationsstufe (11) erfolgt die destillative Abtrennung eines Destillationsrückstandes (P), der das Verfahrensprodukt darstellt und in Tank (15) gesammelt wird.

Die Destillationsstufe (11) kann beispielsweise aus einem einstufigen Verdampfer bestehen, der neben dem Destillationsrückstand (P) ein Destillat (O) liefert.

Das Destillat (O) enthält neben Anilin das gesamte hydrophobe Lösungsmittel aus (N) und wird gegebenenfalls nach Zusatz von Frischanilin (Q) als Extraktionsmittel (M) verwendet.

Die die Produktextraktionsstufe (8) verlassende wäßrige Phase (G) enthält nur noch sehr geringe Anteile von unter 5 Gew.-%, vorzugsweise von unter 2 Gew.-%, an Verfahrensprodukten (Anilin/Formaldehyd-Kondensate).

Mit der wäßrigen Phase (G) in Fig. 2 bzw. (H) in Fig. 1 wird in der mehrstufig arbeitenden Nachextraktionsstufe (10), die im allgemeinen bei Temperaturen von 40°C - 110°C betrieben wird, die organische Phase (E) aus dem Scheider (7) extrahiert, wobei in (E) enthaltene Verfahrensprodukte gegen Anilin ausgetauscht und in die wäßrige Phase (J) in Fig. 2 bzw. (C) in Fig. 1 überführt werden, so daß bei der 1. Variante des Verfahrens eine organische Phase (L) resultiert, die an Kondensationsprodukten verarmt ist.

Die Restgehalt an Verfahrensprodukten in (B) beträgt in der Regel < 10 Gew.-%, vorzugsweise < 5 Gew.-%. In diesem Rest an Kondensationsprodukten liegt u.a. das 2,2'-Isomere des Diaminodiphenylmethans in relativ angereicherter Form vor.

Ebenso verbleiben die gegebenenfalls am Ende der Umlagerungsreaktion in (6) im Reaktionsgemisch noch vorliegenden Kondensationszwischenprodukte zunächst bei der Phasentrennung im Scheider (7) in der vorliegenden Ausführungsform nahezu quantitativ in der organischen Phase (E), in der sie sich beim Durchlaufen der Nachextraktionsstufe (10) in der Restfraktion aufkonzentrieren und anschließend mit dem Mengenstrom (B) rezykliert werden.

Die in der Nachextraktionsstufe (10) anfallende organische Phase (L) wird in die Aminalstufe (3) zurückgeführt.

Die in der Nachextraktionsstufe (10) in Fig. 1 bzw. (9) in Fig. 2 anfallende wäßrige Phase (C) wird in den Reaktionsprozess zwischen dem Beginn der ersten Reaktionsstufe (5) und vor Ende der zweiten Reaktionsstufe (6) zurückgeführt.

Vorzugsweise wird bei der Durchführung der 1. Variante des erfindungsgemäßen Verfahrens zwischen der Produktextraktionsstufe (8) und der Nachextraktionsstufe (10) der bereits früher erwähnte Wasserverdampfer angeordnet.

In dem Wasserverdampfer (9) wird der wäßrigen Lösung (G in Fig. 1 bzw. J in Fig. 2) eine Wassermenge (K) entzogen, die im allgemeinen bis zu 80 Gew.-%, vorzugsweise jedoch weniger als 50 Gew.-%, der Wassermenge der in den Verdampfer eingebrachten wäßrigen Phase ausmacht. Die Wassermenge (K) wird vorzugsweise zwischen der zweiten Reaktionsstufe (6) und der Produktextraktionsstufe (8) in das Reaktionsgemisch zurückgeführt (K'), gegebenenfalls vorher jedoch zumindest teilweise zum Waschen der die Produktextraktionsstufe (8) verlassenden organischen Phase zwecks Entfernung von Saurespuren (K'') und/oder zum Waschen der an den Prozessanfang zurückzuführenden organischen Phase (K''') verwendet, wobei in den betreffenden Waschstufen (12) bzw. (13) die wäßrigen Phasen (D') bzw. (D'') resultieren.

Bei dieser Arbeitsweise (Entfernung von Wasser in (9) und Rückführung) wird die Umlagerung in den Reaktoren (5) und (6) bei einem geringeren Wassergehalt der wäßrigen Phase durchgeführt als die Extraktion in der Extraktionsstufe (8).

Die 2. Variante des erfindungsgemäßen Verfahrens besteht darin, auf die Aminalstufe (3) teilweise oder vollständig zu verzichten. In der Praxis bedeutet dies, daß eine Teilmenge des in der Reaktion eingesetzten Gemischs aus Arylamin und hydrophobem Lösungsmittel (B') und/oder eine Teilmenge des eingesetzten wäßrigen Formaldehyds (A') nicht in die Aminalstufe (3) sondern vor bzw. in die erste Reaktionsstufe (5) geleitet werden. Im Extremfall (völliger Verzicht auf eine Aminalvorstufe) kann auch die Gesamtmenge des Gemischs aus Anilin und hydrophobem Lösungsmittel und die Gesamtmenge des wäßrigen Formaldehyds direkt zur ersten Reaktionsstufe (5) geleitet werden. Bei Verzicht auf eine Aminalvorstufe muß jedoch, wie oben ausgeführt, die Reaktion auf jeden Fall zweistufig unter Verwendung der Reaktionsstufen (5) und (6) durchgeführt werden. Bei einer solchen Arbeitsweise wird naturgemäß das eingebrachte und durch die Kondensation entstehende Wasser nur teilweise oder überhaupt nicht über den Phasenscheider (4) entfernt. Die Entfernung dieses Wassers geschieht dann beispielsweise aus dem Destillat aus (9) als Mengenstrom K^{IV}, der direkt in den Abwassertank (14) geleitet wird.

Gemäß einer 3. Variante können die Reaktionsstufen (5) und (6) zu einer einzigen Reaktionsstufe zusammengefaßt werden, die dann unter den obengenannten Bedingungen bezüglich der Reaktionstemperatur und der Reaktionszeit betrieben wird. Im Falle einer einstufigen Reaktionsführung ist jedoch auf jeden Fall eine vorgeschaltete Aminalstufe (3) erforderlich.

Gemäß einer 4., mit der 1. und 2. Variante kombinierbaren Variante erfolgt in der ersten Reaktionsstufe die Umsetzung in Gegenwart von lediglich einem Teil (C') der zurückgeführten Katalysatorlösung und unter Zugabe der verbleibenden Menge an Katalysatorlösung (C'', C''' und/oder C'''') hinter der ersten Reaktionsstufe (5) und vor der Produktextraktionsstufe (8). Vorzugsweise wird gemäß dieser 4. Variante so gearbeitet, daß man den Katalysatorstrom (C) in zwei Teilströme (C') und (C'') auftrennt und den ersten Teilstrom (C') in die erste Reaktionsstufe (5) und den zweiten Teilstrom (C'') im weiteren Verlauf der ersten Reaktionsstufe (5) und vor bzw. in der zweiten Reaktionsstufe (6) in das Reaktionsgemisch einleitet. Das Gewichtsverhältnis zwischen organischer Phase in (5) und zunächst zugeführter wäßriger Phase (C') z.B. im ersten Rührkessel von (5) liegt zwischen 1:1 und 100:1, vorzugsweise zwischen 3:1 und 30:1.

Besonders bevorzugt wird gemäß dieser 4. Variante so gearbeitet, daß man als ersten Katalysatorteilstrom den Mengenstrom (X) insgesamt oder einen Teil (X') davon in der ersten Reaktionsstufe einsetzt (5). (X) wird als Teilstrom der die Hauptextraktionsstufe (8) verlassenden wäßrigen Phase entnommen - vor oder nach Durchlaufen der Konzentrierstufe (9) - und enthalt verfahrensgemäß sehr wenig Kondensationsprodukte. Als zweiter Katalysatorteilstrom wird die Nachextraktionsstufe (9) verlassende wärßige Phase (C) eingesetzt, entweder in ihrer Gesamtheit, dann ist (C) gleichbedeutend mit (C''), oder gegebenenfalls nach Abzweigen einer Teilmenge (Y) von (C). (C'') wird nach Zugabe von (X), aber vor Beendigung der 2. Umlagerung in (6) dem Reaktionsgemisch zugesetzt. (Y) wird nach beendeter 2. Umlagerung in (6), aber vor Eintritt in die Hauptextraktionsstufe (8) zugesetzt.

Gemeinsam ist den beschriebenen Varianten des erfindungsgemäßen Verfahrens, daS es in der Nachextraktionsstufe (10) vorzugsweise zu einer weitgehend selektiven Auftrennung der in (E) enthaltenen Reaktionsprodukte, d.h. der am Ende des eigentlichen Reaktionsteils (6) vorliegenden und in Scheider (7) mit (E) abgetrennten Fraktion der Reaktionsprodukte kommt in:
1. eine in der die Nachextraktionsstufe (10) verlassenden organischen Phase verbleibende Restfraktion. In dieser Restfraktion sind die bereits in (E) gegenüber (F) angereicherten Bestandteile wie Kondensationszwischenprodukte vom N-Aminobenzylamintyp, gegebenenfalls vom Aminaltyp, Kondensationsendprodukte wie 2,2'-Diaminodiphenylmethan, gegebenenfalls 2,4'-Diaminodiphenylmethan und gegebenenfalls andere nicht näher spezifizierte Zwischen-, End- und/oder Nebenprodukte - aufkonzentriert und werden mit der organischen Phase (B) an den Anfang des Reaktionsgeschehens recyclisiert,
2. eine Produktfraktion, die sich in der wäßrigen Phase (F) befindet und in der die unter 1. aufgeführten Produktkomponenten relativ abgereichert sind. Verfahrensgemäß wird diese wäßrige Phase in den Reaktionsablauf vor beendeter 2. Umlagerung in (6) recyclisiert, wodurch insbesondere das in (C) enthaltene 2,4'-Diaminodiphenylmethan zu höherkernigen Homologen weiterreagieren kann.
   Gegebenenfalls wird ein Teilstrom (Y) von (C) abgetrennt und mit dem Reaktionsgemisch erst nach beendeter Umlagerung (6) vorzugsweise unmittelbar vor Eintritt der wäßrigen Phase (F) in die Produktextraktionsstufe (8) mit der wäßrigen Phase (F) vereinigt, um das in dem Teilstrom (Y) enthaltene 2,4'-Isomere zu erhalten und gezielt dem Verfahrens-Endprodukt zuzuführen.

Bei Beachtung dieser Bedingungen resultieren Verfahrensprodukte, bei denen das Gewichtsverhältnis von 2,2'-Diaminodiphenylmethan zu 2,4'-Diaminodiphenylmethan in der Regel unter 1 : 20 liegt.

Grundsätzlich sollte bei allen Varianten die der Destillationsstufe (11) zugeführte organische Phase aus (8) praktisch frei von Säurespuren sein. Dies kann durch intensives Waschen in der Waschstufe (12) und/oder durch Neutralisation in einer Neutralisationsstufe (nicht gezeichnet) erfolgen.

Die nachfolgenden Beispiele dienen der weiteren Erläuterung des erfindungsgemäßen Verfahrens:

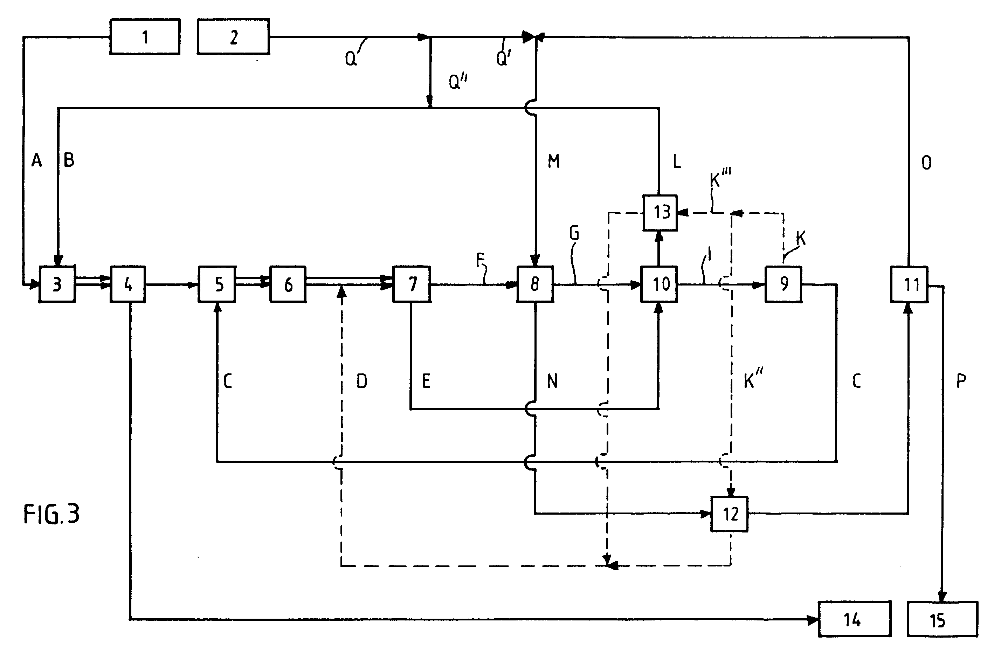

### Beispiel 1 (Fig. 3):

In einem aus zwei hintereinander geschalteten Rührkesseln bestehenden Reaktor (3) wird 30 %ige wäßrige Formalinlösung (Mengenstrom A) mit einem Anilin-Xylolgemisch, welches noch Polyarylanteile enthält (Mengenstrom B), bei 40°C zur Reaktion gebracht.
(A) 0,52 kg/h Formaldehyd
   1,22 kg/h Wasser
(B) 0,22 kg/h Polyarylamin
   3,77 kg/h Anilin
   3,02 kg/h ortho-Xylol
In dem anschließenden Scheider (4) wird die untere, wäßrige Phase als Abwasser abgetrennt und in dem Abwassertank (14) gesammelt.

Die obere organische Phase wird in einem zweiten, aus drei Rührkesseln bestehenden Reaktor (5) übergeführt und dort mit dem Mengenstrom (C), der den sauren Katalysator enthält, vermischt.
(C) 1,51 kg/h Polyarylamin
   1,30 kg/h Anilin
   0,47 kg/h Chlorwasserstoff
   2,80 kg/h Wasser
Die gemessenen und geregelten Temperaturen in den drei Kesseln des Reaktors (5) betragen 30°C, 40°C und 60°C.

In einem weiteren, ebenfalls aus drei Rührkesseln bestehenden Reaktor (6) werden die Temperaturen 100°C, 135°C und 140°C durchlaufen, die durch Aufheizen unter dem sich einstellenden Eigendruck des Systems eingestellt werden.

Nach dem Abkühlen des Reaktionsgemisches auf 95°C und Entspannen auf Normaldruck und nach Zugabe des HCl-Waschwassers aus den Extraktiansstufen (12) und (13) werden im Phasenscheider (7) die organische Phase (Mengenstrom E) und die wäßrige Phase (Mengenstrom F) voneinander getrennt.
(E) 1,68 kg/h Polyarylamin
   1,10 kg/h Anilin
   3,02 kg/h ortho-Xylol
(F) 2,95 kg/h Polyarylenamin
   1,30 kg/h Anilin
   0,47 kg/h Chlorwasserstoffsäure
   4,61 kg/h Wasser
Die wäßrige Phase (F) wird anschließend in der Extraktionskolonne (8) im Gegenstrom mit einem eingestellten Anilin-Xylolgemisch (Mengenstrom M) kontinuierlich extrahiert.
(M) 9,0 kg/h Anilin
   7,5 kg/h ortho-Xylol
und geht dabei über in die an Polyarylamin verarmte wäßrige Phase Mengenstrom (G).
(G) 0,44 kg/h Polyarylamin
   1,87 kg/h Anilin
   0,47 kg/h Chlorwasserstoffsäure
   4,61 kg/h Wasser
Der Mengenstrom (G) wird in einer weiteren Extraktionskolonne (10) zur Gegenstromextraktion der in (7) abgetrennten organischen Phase (E) eingesetzt.

Der in (10) resultierende, gegenüber dem eingesetzten wäßrigen Mengenstrom (G) an Polyarylamin angereicherte Mengenstrom (I) wird in der Destillationsstufe (9) aufkonzentriert und unter Entnahme von Destillat (Mengenstrom K) anschließend als Mengenstrom (C) in den Reaktor (5) zurückgeführt.
(I) 1,51 kg/h Polyarylamin
   1,30 kg/h Anilin
   0,47 kg/h Chlorwasserstoffsäure
   4,61 kg/h Wasser
Die in der Extraktionsstufe (10) resultierende an Polyarylamin gegenüber (D) verarmte organische Phase (L) wird nach Zugabe von Anilin (Q'') als Mengenstrom (B) in den Reaktor (3) zurückgeführt.

Die in der Produktextraktion (8) und (10) anfallenden das Reaktionsprodukt enthaltenden Phasen (Mengenstrom N) werden in den Waschstufen (12) bzw. (13) mit dem im wesentlichen aus Wasser bestehenden Destillat der Destillationsstufe (9) (Mengenstrom K) extrahiert.
(N) 2,90 kg/h Polyarylamin
   8,43 kg/h Anilin
   7,5 kg/h ortho-Xylol
(K) 1,2 kg/h Wasser
In der Waschstufe (12) wird der HCl-Gehalt des Mengenstroms (N), der bei ca. 0,2 Gew.-% liegt, unter den angegebenen Bedingungen reduziert auf < 0,01 Gew.-%.

Das HCl-haltige Waschwasser wird zusammen mit dem Waschwasser der Stufe (13) als Mengenstrom (D) in das Reaktionsgemisch hinter Stufe (6) und vor der Stufe (7) recyclisiert.

Die die Waschkolonne (12) verlassende organische Phase wird nach Entfernung restlicher Säurespuren durch Neutralisation mit überschüssiger Natronlauge und Abtrennung des gebildeten Kochsalzes und der unverbrauchten Natronlauge in einer Destillationsstufe (11) aufgetrennt in ein Destillat (Mengenstrom O) und einen Destillationsrückstand (Mengenstrom P).
(O) 8,43 kg/h Anilin
   7,5 kg/h ortho-Xylol
(P) 2,9 kg/h Polyarylamin
Durch Zugabe von frischem Anilin (Q') aus dem Vorratsbehälter (2) zum Destillat (O) wird das in (8) benötigte Extraktionsmitel nach Menge und Zusammensetzung eingestellt (Mengenstrom M).

Der Destillationsrückstand (Mengenstrom P) der Destillationsstufe (11) hat folgende Zusammensetzung:
0,2 Gew.-% 2,2'-Diaminodiphenylmethan
4,7 Gew.-% 2,4'-Diaminodiphenylmethan
50,4 Gew.-% 4,4'-Diaminodiphenylmethan
0,2 Gew.-% N-substituierte Diaminodiphenylmethane
22,0 Gew.-% Triamin
10,8 Gew.-% Tetramin
ca. 11,7 Gew.-% höher als tetrafunktionelle Polyamine

## Patentansprüche

1. Verfahren zur Herstellung von mehrkernigen aromatischen Polyaminen durch Umsetzung von Anilin mit Formaldehyd in Gegenwart von Wasser und sauren Katalysatoren in einer ein- oder zweistufigen Reaktion innerhalb des Temperaturbereichs von 0 bis 180°C, gegebenenfalls unter Vorschaltung einer Aminal-Vorstufe, in welcher die Bildung von N,N'-disubstituiertem Aminal stattfindet, welches dann ein- oder mehrstufig in Gegenwart von Säurekatalysator innerhalb des Temperaturbereichs von 0 bis 180°C in das gewünschte Endprodukt überführt wird, Aufarbeitung des resultierenden Reaktionsgemischs und Gewinnung der resultierenden Verfahrensendprodukte durch Extraktion mit einem Anilin-haltigen hydrophober Lösungsmittel in einer Produktextraktionsstufe (8) destillative Auftrennung der hierbei anfallenden organischen Phase in (i) ein aus Anilin-haltigem Lösungsmittel bestehendes Destillat, welches, gegebenenfalls nach Zugabe von Frischanilin erneut bei der Extraktion eingesetzt wird und (ii) einen im wesentlichen aus Verfahrensendprodukt bestehenden Destillationsrückstand und Rückführung von den Säurekatalysator enthaltender wäßriger Phase unter Wiederverwendung des in ihr enthaltenen Katalysators, unter Entfernung des bei der Kondensationsreaktion entstehenden Kondensationswassers und des in das System mit der wäßrigen Lösung des Formaldehyds eingebrachten Wassers in einem der Aminalvorstufe nachgeschalteten und der ersten Reaktionsstufe vorgeschalteten Wasserabscheider und/oder in einem der Extraktionsstufe nachgeschalteten Verdampfer, dadurch gekennzeichnet, daß man
a) den in Form einer wäßrigen Lösung zum Einsatz gelangenden Formaldehyd in einer Aminal-Vorstufe (3) mit einer aus Anilin, gegebenenfalls Anilin-Formaldehyd-Kondensationsprodukten und aus hydrophobem Lösungsmittel bestehenden organischen Phase und/oder in der ersten Reaktionsstufe (5) mit einer aus Anilin, gegebenenfalls Anilin-Formaldehyd-Kondensationsprodukten und aus hydrophobem Lösungsmittel bestehenden organischen Phase und recyclisierter, den Katalysator in Form von Aminsalzen enthaltender wäßriger Phase durch Vermischen zur Reaktion bringt,
b) das so erhaltene zweiphasige Reaktionsgemisch nach Beendigung der Reaktion in einem der Produktextraktionsstufe (8) vorgeschalteten Phasenscheider (7) in eine wäßrige Phase und eine organische Phase auftrennt,
c) die im Phasenscheider (7)anfallende organische Phase in einer der Produktextraktionsstufe (8) nachgeschalteten Nachextraktionsstufe (10) mit wenigstens einem Teil der in der Produktextraktionsstufe (8) anfallenden, weitgehend vom Reaktionsprodukt befreiten wäßrigen Phase extrahiert,
d) die in der Nachextraktionsstufe (10) anfallende mit Reaktionsprodukt der im Phasenscheider (7) anfallenden organischen Phase angereicherte wäßrige Phase vollständig oder zumindest teilweise in den Reaktionsprozess vor beendeter Umlagerung (6) zurückführt und den gegebenenfalls verbleibenden Rest der wäßrigen Phase zwischen die Umlagarungsstufe (6) und die Hauptextraktionsstufe (8) zurückführt,
e) die in der Nachextraktionsstufe (10) anfallende, aus Anilin, gegebenenfalls Anilin-Formaldehyd-Kondensationsprodukten und aus hydrophobem Lösungsmittel bestehende organische Phase an den Prozessanfang zurückführt und gemäß a) umsetzt,
f) die im Phasenscheider (7) anfallende wäßrige Phase in der Produktextraktionsstufe (8) mit Anilin-haltigem, hydrophoben Lösungsmittel extrahiert,
g) die in der Produktextraktionsstufe (8) anfallende wäßrige Phase vollständig oder zumindest teilweise der Nachextraktionsstufe (10) zuführt und gegebenenfalls den verbleibenden Rest vor die Umlagerungsstufe (6), vorzugsweise an den Prozeßanfang zurückführt,
h) die in der Produktextraktionsstufe (8) anfallende organische Phase in der Destillationsstufe (11) in ein aus Anilin-haltigem hydrophoben Lösungsmittel bestehendes Destillat und einen im wesentlichen aus Verfahrensendprodukt bestehenden Destillationsrückstand auftrennt und
i) das in der Destillationsstufe (11) anfallende Destillat gegebenenfalls nach Zugabe von Frischanilin in der Produktextraktionsstufe (8) als Extraktionsmittel verwendet.

2. Verfahren gemäß Anspruch 1, dadurch gekennzeichnet, daß man wenigstens einen Teil der die Produktextraktionsstufe (8) verlassenden wäßrigen Phase vor oder nach Durchlaufen der Nachextrationsstufe (10) in einer Destillationsstufe (9) destillativ eine Wassermenge entzieht, die über die Wassermenge hinausgeht, die zur Aufrechterhaltung eines konstanten Wasservolumens im Kreislauf notwendig ist und diese über die zur Konstanthaltung des Wasservolumens hinausgehende Wassermenge an einer beliebigen Stelle hinter der letzten Reaktionsstufe (6) und vor der Extraktionsstufe (8) in den Kreislauf zurückführt.

3. Verfahren gemäß Anspruch 2, dadurch gekennzeichnet, daß man das in der Destillationsstufe (9) als Destillat anfallende Wasser vor seiner Rückführung zumindest teilweise zum Waschen der die Produktextraktionsstufe (8) verlassenden organischen Phase und/oder der die Nachextraktionsstufe (10) verlassenden organischen Phase verwendet.

4. Verfahren gemaß Anspruch 1 bis 3, dadurch gekennzeichnet, daß man die an den Prozeßanfang zurückzuführende Katalysatorlösung (C) in zwei oder mehrere Teilströme aufteilt und den ersten Teilstrom in die erste Reaktionsstufe (5) und die anderen Teilströme im weiteren Verlauf der ersten Reaktionsstufe (5) und vor der Produktextraktionsstufe (8) in das Reaktionsgemisch zurückleitet.

## Claims

1. A process for the production of polynuclear aromatic polyamines by reaction of aniline with formaldehyde in the presence of water and acidic catalysts in one or two stages at a temperature in the range from 0 to 180°C, optionally with inclusion of a preliminary aminal stage in which N,N'-disubstituted aminal is formed and is then converted into the desired end product in one or more stages in the presence of acid catalyst at a temperature in the range from 0 to 180°C, working up of the resulting reaction mixture and recovery of the resulting end products by extraction with an aniline-containing hydrophobic solvent in a product extraction stage (8), separation of the organic phase accumulating by distillation into (i) a distillate consisting of aniline-containing solvent, which is reused in the extraction stage, optionally after addition of fresh aniline, and (ii) a distillation residue consisting essentially of end product and recycling of aqueous phase containing the acid catalyst with reuse of the catalyst present therein and with removal of the water of condensation formed in the condensation reaction and the water introduced into the system with the aqueous formaldehyde solution in a water separator following the preliminary aminal stage and preceding the first reaction stage and/or in an evaporator following the extraction stage, characterized in that
a) the formaldehyde used in the form of an aqueous solution is reacted by mixing in a preliminary aminal stage (3) with an organic phase consisting of aniline, optionally aniline/formaldehyde condensates and hydrophobic solvent and/or in a first reaction stage (5) with an organic phase consisting of aniline, optionally aniline/formaldehyde condensates and hydrophobic solvent and recycled aqueous phase containing the catalyst in the form of amine salts,
b) the two-phase reaction mixture obtained is separated on completion of the reaction into an aqueous phase and an organic phase in a phase separator (7) preceding the product extraction stage (8),
c) the organic phase accumulating in the phase separator (7) is extracted in a post-extraction stage (10) following the product extraction stage (8) with at least part of the aqueous phase substantially freed from reaction product which accumulates in the product extraction stage (8),
d) the aqueous phase accumulating in the post-extraction stage (10), which is enriched with reaction product of the organic phase accumulating in the phase separator (7), is completely or at least partly recycled to the reaction before the completion of rearrangement (6) and any aqueous phase remaining is recycled between the rearrangement stage (6) and the main extraction stage (8),
e) the organic phase consisting of aniline, optionally aniline/formaldehyde condensates and hydrophobic solvent, which accumulates in the post-extraction stage (10), is returned to the beginning of the process and reacted in accordance with a),
f) the aqueous phase accumulating in the phase separator (7) is extracted with aniline-containing hydrophobic solvent in the product extraction stage (8),
g) the aqueous phase accumulating in the product extraction stage (8) is delivered completely or at least partly to the post-extraction stage (10) and any residue remaining is preferably recycled to the beginning of the process before the rearrangement stage (6),
h) the organic phase accumulating in the product extraction stage (8) is separated in the distillation stage (11) into a distillate consisting of aniline-containing hydrophobic solvent and a distillation residue consisting essentially of end product and
i) the distillate accumulating in the distillation stage (11) is used as extractant in the product extraction stage (8), optionally after addition of fresh aniline.

2. A process as claimed in claim 1, characterized in that, before or after the post-extraction stage (10), water is removed by distillation from at least part of the aqueous phase leaving the product extraction stage (8) in a quantity exceeding the quantity of water required to maintain a constant volume in the circuit and this quantity of water exceeding the quantity required to maintain a constant water volume is returned to the circuit at any point after the last reaction stage (6) and before the extraction stage (8).

3. A process as claimed in claim 2, characterized in that, before it is recycled, the water accumulating as distillate in the distillation stage (9) is at least partly used to wash organic phase leaving the product extraction stage (8) and/or the organic phase leaving the post-extraction stage (10).

4. A process as claimed in claims 1 to 3, characterized in that the catalyst solution C) to be returned to the beginning of the process is divided into two or more partial streams of which the first partial stream is returned to the first reaction stage (5) while the other partial streams are returned to the reaction mixture in the further course of the first reaction stage (5) and before the product extraction stage (8).

## Revendications

1. Procédé pour la préparation de polyamines aromatiques polynucléaires par réaction d'aniline avec du formaldéhyde en présence d'eau et de catalyseurs acides dans une réaction à une ou deux étapes à l'intérieur du domaine de températures de 0 à 180°C, en insérant éventuellement une pré-étape d'aminal, dans laquelle a lieu la formation d'un aminal N,N'-disubstitué, lequel est transformé alors en produit final souhaité en une ou plusieurs étapes en présence de catalyseur acide à l'intérieur du domaine de températures de 0 à 180°C, par traitement du mélange réactionnel résultant et récupération des produits finaux du procédé résultants par extraction avec un solvant hydrophobe contenant de l'aniline dans une étape d'extraction de produit (8) de séparation par distillation de la phase organique ainsi produite en (i) un distillat constitué de solvant contenant de l'aniline, lequel est éventuellement introduit à nouveau dans l'extraction après addition d'aniline fraîche et en (ii) un résidu de distillation constitué essentiellement du produit final du procédé et par recyclage d'une phase aqueuse contenant le catalyseur acide en réutilisant le catalyseur contenu dans celle-ci, en séparant l'eau de condensation formée dans la réaction de condensation et l'eau apportée dans le système avec la solution aqueuse du formaldéhyde dans un séparateur d'eau disposé en amont de la première étape de réaction et en aval de la pré-étape d'aminal et/ou dans un évaporateur monté en aval de l'étape d'extraction, caractérisé en ce que :
a) on fait réagir en mélangeant le formaldéhyde introduit sous la forme d'une solution aqueuse dans une pré-étape d'aminal (3) avec une phase organique constituée d'aniline, éventuellement de produits de condensation aniline-formaldéhyde et de solvant hydrophobe et/ou dans la première étape de réaction (5) avec une phase organique constituée d'aniline, éventuellement de produits de condensation aniline-formaldéhyde et de solvant hydrophobe et une phase aqueuse recyclée contenant le catalyseur sous la forme de sels d'amines,
b) on sépare le mélange réactionnel biphasé ainsi obtenu après la fin de la réaction dans un séparateur de phases (7) disposé en amont de l'étape d'extraction de produit (8) en une phase aqueuse et une phase organique,
c) on extrait la phase organique produite dans le séparateur de phases (7) dans une étape d'extraction subséquente (10) disposée en aval de l'étape d'extraction de produit (8) avec au moins une partie de la phase aqueuse produite dans l'étape d'extraction de produit (8), largement exempte de produit de réaction,
d) on renvoie complètement ou au moins partiellement la phase aqueuse produite dans l'étape d'extraction subséquente (10) enrichie avec du produit de réaction de la phase organique produite dans le séparateur de phases (7) dans le procédé de réaction avant la fin de la transposition (6) et on renvoie le résidu éventuellement restant de la phase aqueuse entre l'étape de transposition (6) et l'étape principale d'extraction (8),
e) on renvoie la phase organique produite dans l'étape d'extraction subséquente (10) constituée d'aniline, éventuellement de produits de condensation aniline-formaldéhyde et de solvant hydrophobe au début du procédé et on la fait réagir selon a),
f) on extrait la phase aqueuse produite dans le séparateur de phases (7) dans l'étape d'extraction de produit (8) avec du solvant hydrophobe contenant de l'aniline,
g) on envoie complètement ou au moins partiellement la phase aqueuse produite dans l'étape d'extraction de produit (8) dans l'étape d'extraction subséquente (10) et on renvoie éventuellement le résidu restant avant l'étape de transposition (6), de préférence au début du procédé,
h) on sépare la phase organique produite dans l'étape d'extraction de produit (8) dans l'étape de distillation (11) en un distillat constitué de solvant hydrophobe contenant de l'aniline et un résidu de distillation constitué essentiellement de produit final de procédé et
i) on utilise comme agent d'extraction dans l'étape d'extraction de produit (8) le distillat produit dans l'étape de distillation (11), éventuellement après addition d'aniline fraîche.

2. Procédé selon la revendication 1, caractérisé en ce qu'on prélève au moins une partie de la phase aqueuse quittant l'étape d'extraction de produit (8) avant ou après le passage dans l'étape d'extraction subséquente (10) dans une étape de distillation (9) par distillation d'une quantité d'eau, en excès par rapport à la quantité d'eau qui est nécessaire pour maintenir un volume d'eau constant dans le circuit fermé et en ce qu'on renvoie cette quantité d'eau en excès par rapport à celle qui maintient le volume d'eau constant à un endroit quelconque après la dernière étape de réaction (6) et avant l'étape d'extraction (8) dans le circuit fermé.

3. Procédé selon la revendication 2, caractérisé en ce qu'on utilise au moins partiellement l'eau produite en tant que distillat dans l'étape de distillation (9) avant son recyclage pour laver la phase organique quittant l'étape d'extraction de produit (8) et/ou la phase organique quittant l'étape d'extraction subséquente (10).

4. Procédé selon l'une quelconque des revendications 1 à 3, caractérisé en ce qu'on répartit la solution de catalyseur (C) à recycler au début du procédé en deux ou plusieurs courants partiels et en ce qu'on recycle dans le mélange réactionnel le premier courant partiel dans la première étape de réaction (5) et les autres courants partiels au cours de la réaction de la première étape de réaction (5) et avant l'étape d'extraction de produit (8).
